(19) Europäisches Patentamt
European Patent Office
Office européen des brevets

(11) Veröffentlichungsnummer: **0 212 608**
**B1**

(12) **EUROPÄISCHE PATENTSCHRIFT**

(45) Veröffentlichungstag der Patentschrift: **01.08.90**

(51) Int. Cl.[5]: **C 07 C 323/12,** C 07 C 317/18

(21) Anmeldenummer: **86111388.4**

(22) Anmeldetag: **18.08.86**

(54) **Verfahren zur Herstellung von Halogenphenyl-oxethylsulfiden und deren Oxidationsprodukten.**

(30) Priorität: **28.08.85 DE 3530709**

(43) Veröffentlichungstag der Anmeldung:
**04.03.87 Patentblatt 87/10**

(45) Bekanntmachung des Hinweises auf die Patenterteilung:
**01.08.90 Patentblatt 90/31**

(84) Benannte Vertragsstaaten:
**CH DE FR GB IT LI**

(56) Entgegenhaltungen:
**EP-A-0 092 909**
**EP-A-0 094 821**
**DE-A-2 419 039**
**DE-A-3 135 367**
**US-A-4 297 513**

**PATENT ABSTRACTS OF JAPAN, unexamined applications, C field, vol. 8, no. 7, 12. Januar 1984 THE PATENT OFFICE JAPANESE GOVERNMENT Seite 42 C 204**

(73) Patentinhaber: **HOECHST AKTIENGESELLSCHAFT**
**Postfach 80 03 20**
**D-6230 Frankfurt am Main 80 (DE)**

(72) Erfinder: **Papenfuhs, Theodor, Dr.**
**Heinrich-Bleicher-Strasse 40**
**D-6000 Frankfurt am Main 50 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 212 608 B1

**Beschreibung**

Die Erfindung betrifft ein vorteilhaftes Verfahren zur Herstellung von Halogenphenyl-oxyethylsulfiden und deren Oxidationsprodukten (Halogenphenyl-oxethylsulfoxiden und Halogenphenyl-oxethylsulfonen) unter Vermeidung der den bekannten Verfahren anhaftenden Mängel.

Halogenphenyl-oxethylsulfone der allgemeinen Formel (A)

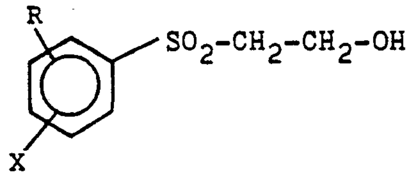

(A)

in welcher R ein Chlor- oder Bromatom, und X ein Wasserstoff-, Chlor- oder Bromatom oder eine Alkyl-$C_1$—$C_6$-gruppe bedeuten, stellen wertvolle Vorprodukte zur Herstellung von Reaktivfarbstoffen der Vinylsulfonreihe dar.

Ihre technische Herstellung erfolgte bischer aus den entsprechenden Sulfochloriden der allgemeinen Formel (B)

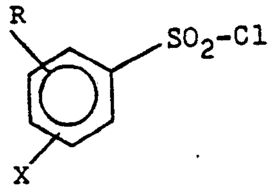

(B)

in welcher R und X die vorstehend genannten Bedeutungen haben, durch Reduktion mit Sulfit in wässrigem Medium und anschließende Umsetzung der hierbei gebildenten Sulfinate mit Ethylenoxid [DE—OS 3 302 647 bzw. EP—OS 01 15 328], wobei die Ausgangsverbindungen der genannten Formel (B) durch Sulfochlorierung geeigneter Halogenbenzolverbindungen [DE—OS 3 302 647] oder durch Umsetzung von Benzoldiazoniumhalogeniden mit Schwefeldioxid oder Alkalihydrogensulfit in Gegenwart von Kupfer oder Kupferverbindungen (Sandmeyer-Reaktion) hergestellt werden [DE—PS 2 308 262 (US—PS 3 947 512)].

Dieses bekannte Verfahren ist technisch unbefriedigend, weil sowohl die erwähnten Verfahren zur Herstellung der Ausgangsverbindungen der Formel (B) als auch die sich daran anschließende Umsetzung der Sulfochloride mit erheblichen Nachteilen behaftet ist. So erfordert die Sulfochlorierung und die Sandmeyer-Reaktion einen erheblichen Aufwand zur Abwasserentsorgung. Die Sulfit-Reduktion ist durch einen hohen Salzanfall belastet und bei der Oxethylierung mittels Ethylenoxid werden aus Gründen der Sicherheit und Gewerbehygiene aufwendige Apparaturen benötigt.

Es bestand daher ein erheblicher Bedarf für ein anderes Verfahren zur Herstellung der Verbindungen der genannten Formel (A), durch welches die Nachteile des erwähnten bekannten technischen Verfahrens vermieden werden und eine wirtschaftlichere Herstellung der Verbindungen der Formel (A) ermöglicht wird.

Aus der Literatur (DE—OS 3 135 367; EP—OS 0 092 909) sind Verfahren bekannt geworden, Nitroaryloxethylsulfide durch Umsetzung von aktivierten Halogen-nitrobenzolen (z.B. o-bzw. p-Nitrochlorbenzol) mit Mercaptoethanol im alkalischen, ggfs. dipolar-aprotische Lösungsmittel enthaltenden Medium zugänglich zu machen, die dann durch Oxidation mit Wasserstoffperoxid in Gegenwart katalytischer Mengen Wolfram(VI)- verbindungen in die als Farbstoffvorprodukte gesuchten Nitroaryloxethylsulfone überführt werden.

Diese Verfahren gelingen jedoch nur bei den genannten stark aktivierten Halogennitrobenzolen, versagen aber beispielsweise bereits bei dem weniger aktivierten m-Nitrochlorbenzol.

Weiterhin ist aus der Literatur (EP—OS 00 94 821) ein Verfahren bekannt geworden, nichtaktivierte Halogenaromaten (algebraische Summe der 6'-Hammettkonstanten zusätzlich zur Austrittsgruppe enthaltener Substituenten $\leqq + 0.455$), z.B. die isomeren Dichlorbenzole, mit Alkylmercaptiden Alkyl-S-Me (Me = Alkalimetallatom) in Gegenwart katalytisch wirkender Kronenether oder Polyglykol(-ether) bei Temperaturen von 170—190°C und Reaktionszeiten bis zu 96 Stunden zu Arylalkyl-thioethern umzusetzen und diese ggf. auch zu den entsprechenden Sulfonen zu oxidieren. Dieses Verfahren ist aus den nachstehenden Gründen ungeeignet, entsprechende nicht aktivierte Halogenaromaten der vorstehend genannten Definition (vgl. im Detail EP—OS 00 94 821, Seiten 7—16) technisch mit Mercaptoethanol zu den gesuchten Halogenphenyloxethylsulfiden umzusetzen:

1. Es gelingt nicht, Mercaptoethanol mit Alkalimetallhydroxid umzusetzen und durch geeignetes Aufarbeiten das geforderte wasserfreie Alkalimetallsalz des Mercaptoethanols herzustellen, da dieses sich bereits unter Entwässerungsbedingungen irreversibel zersetzt.

2. Die benötigen Reaktionstemperaturen für den Halogenaustausch von >170°C bedingen eine sehr rasche Veränderung des Mercaptoethanol(-salzes), z.B. Bildung von telomeren bzw. polymeren Ethylensulfid-Derivaten bis zur Verharzung, sowie des ggf. entstehenden Zielproduktes (Wasserabspaltung zu Halogenphenylvinylsulfid).

Es wurde nun überraschenderweise gefunden, daß man Verbindungen der allgemeinen Formel (1)

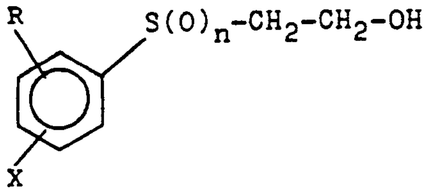

(1)

in welcher R ein Chlor- oder Bromatom, X ein Wasserstoff-, atom oder eine Alkyl-$C_1$—$C_6$-gruppe bedeuten und n die Zahl 0, 1 oder 2 darstellt, in guten Ausbeuten herstellen kann, indem man nichtaktivierte Halogenbenzole der allgemeinen Formel (2)

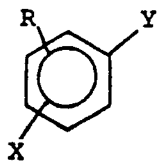

(2)

in welcher R und X die vorstehend genannten Bedeutungen haben und Y ein Chlor- oder Bromatom darstellt, mit Mercaptoethanol in Gegenwart eines Alkalimetall-, beispielsweise eines Lithium-, Natrium- oder Kaliumoxids, -hydroxids oder -carbonats, und in Gegenwart von Polyglykolen, Polyglykolethern oder macrocyclischen Polyethern (Kronenethern) bei Temperaturen von 80°C bis 140°C, vorzugsweise 110°C bis 130°C, umsetzt zu den Halogenphenyl-oxethylsulfiden der allgemeinen Formel (3)

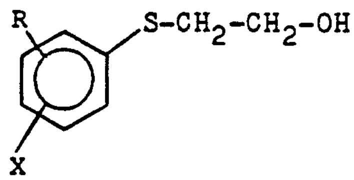

(3)

worin R und X die vorstehend genannten Bedeutungen haben, und diese direkt in der angefallenen Reaktionsmischung oder nach Abtrennung der flüchtigen Bestandteile durch Destillation oder Extraktion und Abtrennung der festen Bestandteile durch Filtration oder Extraktion, vorzugsweise in Gegenwart von Wasser, bei einem pH-Wert von 1 bis 7, vorzugsweise von 3,5 bis 6,0, bei Temperaturen von 20°C bis 120°C, vorzugsweise von 70°C bis 100°C, mit 1 Mol Oxidationsmittel (pro Mol Halogenphenyl-oxethylsulfid) umsetzt zu den Verbindungen der Formel (1) mit n = 1, oder mit mindestens 2 Mol Oxidationsmittel (pro Mol Halogenphenyl-oxethylsulfid) umsetzt zu den Verbindungen der Formel (1) mit n = 2.

Erfindungsgemäß einsetzbare Ausgangsverbindungen der genannten allgemeinen Formel (2) sind beispielsweise die isomeren Dichlorbenzole, und Dichlortoluole, sowie die entsprechenden Bromderivate, wobei die Dichlorbenzole, insbesondere als o- und p-Dichlorbenzol, bevorzugt sind.

Als Alkalimetallverbindungen sind vor allem die Hydroxide, Oxide und Carbonate von Lithium, Natrium und Kalium zu nennen, wobei die Kaliumverbindungen insbesondere als Kaliumhydroxid bevorzugt sind. Ihre Zugabe kann in fester Form oder in Form konzentrierter wäßriger Lösungen oder Suspensionen erfolgen.

Als Polyglykole oder Polyglykolether kommen offenkettige lineare Polymere des Ethylenoxids und deren Alkylether mit der Summenformel

$$R—(OCH_2—CH_2)_n—OR \quad (R = H \text{ oder Alkyl } C_1—C_5;\ n = 3—500)$$

mit einem mittleren Molekulargewicht von 200—20000, vorzugsweise 300—6000, insbesondere 300—2000, und als macrocyclische Polyether (Kronenether), cyclische Polymere des Ethylenoxids mit der Summenformel

$$\left[(O-CH_2-CH_2)_n\right] \quad (n = 4-8)$$

und deren Dicyclohexyl — oder Dibenzo-Homologe, deren Herstellung beispielweise aus US—PS 3 687 978 bekannt ist, in Betracht.

Die Polyglykole und Polyglykolether können in katalytischen Mengen, auf Grund ihrer guten technischen Verfügbarkeit und damit Preiswürdigkeit aber auch in stöchiometrischen oder noch größeren Mengen verwendet werden, wobei sie oft als zusätzliches Lösungsmittel fungieren können. Der Einsatz von Kronenethern muß wegen der schlechten technischen Zugänglichkeit auf möglichst geringe, d.h. katalytische Menge beschränken.

Die beim erfindungsgemäßen Verfahren anfallenden Reaktionsmischungen, welche Polyglykol(ether) oder Kronenether, Alkalimetallhalogenide und ggf. überschüssig eingesetztes Edukt der Formel (2) enthalten, können im Einzelfall direkt zur Oxidation eingesetzt werden. In der Regel ist jedoch eine Abtrennung der flüchtigen Bestandteile, beispielsweise überschüssiges Edukt der Formel (2), durch Destillation oder Extraktion, des Polyglykol(ethers) oder Kronenethers und ggf. des Alkalimetallhalogenids durch Filtration oder Extraktion von Vorteil.

Das dabei resultierende rohe Halogenphenyl-oxethylsulfid der Formel (1) mit n = 0 kann als solches, oder nach Vakuumdestillation in reiner Form zur Oxidation eingesetzt werden, wobei je nach Menge des verwandten Oxidationsmittels und ggf. abgewandelten Oxidationsbedingungen Halogenphenyl-oxethylsulfoxide oder Halogenphenyl-oxethylsulfone resultieren, die von ggf. enthaltenen Begleitsubstanzen (Polyglykol (ether) oder Kronenether, überschüssig eingesetztes Edukt der Formel (2), Alkalimetallhalogenid, Verunreinigungen) im Zuge einer geeigneten Aufarbeitung (beispielsweise Abdestillieren flüchtiger Bestandteile, Lösen des enthaltenen Salzes, Klärfiltration von Verunreinigungen, ggf. nach Zusatz von Absorbentien) befreit und durch Extraktion oder Filtration in reiner Form erhalten werden können.

Das Verfahren wird im einzelnen in der Weise durchgeführt, daß man eine Lösung von Mercaptoethanol in der mindestens äquivalenten, in der Regel einem molaren Überschuß von 5—500%, vorzugsweise 50—200%, entsprechenden Menge Edukt der genannten Formel (2) mit der etwa äquivalenten Menge einer der genannten Alkalimetallverbindungen (wobei ein molarer Unterschuß von maximal 10% bis zu einem molaren Überschuß von maximal 25% möglich ist) versetzt und das Gemisch unter Rühren auf Temperaturen von 80°C—140°C, vorzugsweise von 110°C—130°C erhitzt, wobei das gebildete Wasser, ggf. zusammen mit Anteilen der meist dampfflüchtigen Edukte der Formel (2), bei Normaldruck oder vorzugsweise bei einem der Reaktionstemperatur zur Erzielung eines kräftigen Rückflusses entsprechenden Vakuum abdestilliert und die ggf. mit überdestillierte Menge an Edukt der Formel (2) auskondensiert und, vorzugsweise kontinuierlich, in den Ansatz zurückgeführt wird.

Wenn sich kein wäßriges Destillat mehr abscheidet, was in der Regel nach 4—6 Stunden der Fall ist, wird noch weitere 2—8 Stunden, vorzugsweise 3—6 Stunden, bei Reaktionstemperatur nachgerührt, bis durch analytische Methoden, z.B. Gaschromatografie, kein Mercaptoethanol mehr im Umsetzungsgemisch nachweisbar ist.

Die Zugabe des Polyglykol(ethers) (5—300 Mol%, vorzugsweise 10—200 Mol% des Einsatzes an Edukt der Formel (2) oder des Kronenethers (1—15 Mol%, vorzugsweise 2—5 Mol% des Einsatzes an Edukt der Formel (2)) kann nach Beendigung der Zugabe der Alkalimetallverbindung, oder erst nach der destillativen Abtrennung des Wassers erfolgen, wobei die erstgenannte Verfahrensweise besonders bevorzugt ist, da dann die Bildung des labilen Alkalimetallsalzes des eingesetzten Mercaptoethanols erst im Zuge der Umsetzung unter Entfernen des dabei entstehenden Wassers erfolgt.

Das Umsetzungsgemisch kann in Einzelfällen direkt zur Oxidation eingesetzt werden, vorteilhafter ist es aber, eine Aufarbeitung vorzuschalten. Dazu wird, ggf. nach Abfiltrieren des ausgefallenen Alkalimetallhalogenids, das nicht reagierte Edukt der Formel (2), vorzugsweise im Vakuum, abdestilliert. Das Destillat kann dann, ergänzt um die verbrauchte Menge an Edukt der Formel (2), im nächsten Ansatz wieder eingesetzt werden.

Der Destillations-Sumpf, bestehend aus dem Halogenphenyl-oxyethylsulfid (Formel (1) mit n = o), dem Katalysator, ggf. Alkalimetallhalogenid, und möglicherweise Verunreinigungen, kann durch Vakuumfraktionierung aufgetrennt werden, wobei als Destillat in guter Ausbeute saubere Verbindungen der Formel (1) mit n = 0 erhalten werden. Er kann aber auch als solcher zur Oxidation eingesetzt werden, sofern eine Abtrennung des Katalysators und der Verunreinigungen im Zuge der Aufarbeitung des Oxidates möglich ist.

Zur Oxidation wird das Halogenphenyl-oxyethylsulfid direkt in Form der angefallenen Reaktionsmischung oder nach Abtrennung der flüchtigen und festen Bestandteile, vorzugsweise in Gegenwart von Wasser bei einem pH-Wert von 1—7, vorzugsweise von 3,5—6,0 bei Temperaturen von 20°C—120°C, vorzugsweise von 70°C—100°C, innerhalb von 0,5—5 Stunden, vorzugsweise von 1—3 Stunden, mit dem Oxidationsmittel versetzt und durch 1—5-stündiges, vorzugsweise 2—3-stündiges Nachrühren zu Ende gebracht.

Als Oxidationsmittel kommen Halogene, wie Chlor oder Brom, Hypohalogenite, wie Alkalimetallhypochlorit oder -bromit, und insbesondere Wasserstoffperoxid, vorzugsweise in Gegenwart katalytischer Mengen Wolfram(VI)-verbindungen, wie Wolframtrioxid oder Alkalimetallwolframat, in Frage, wobei der geforderte pH-Wert durch Zugabe von Säuren, vorzugsweise Essigsäure, eingestellt und gehalten wird.

Die Oxidation verläuft exakt stufenweise. Bei Einsatz von 1 Mol Oxidationsmittel pro Mol Halogenphenyl-oxethylsulfid, werden in guter Ausbeute die Halogenphenyl-oxethylsulfoxide der Formel (1) mit n = 1 gebiltet, bei überschüssigem, mindestens 2-molarem Einsatz von Oxidationsmittel entstehen in ebenfalls guter Ausbeute die Halogenphenyl-oxethylsulfone der Formel (1) mit n = 2. Ein größerer Überschuß an

Oxidationsmittel ist zwar möglich, führt aber im Falle der Halogene zu intermediärer Bildung von Halogenphenyl-β-halogenethylsulfonen, die nachfolgend erst wieder zu den Verbindungen der Formel (1) mit n = 2 gespalten werden müssen.

Zur Isolierung der Verbindungen der Formel (1) mit n = 1 oder 2 aus dem angefallenen Reaktionsgemisch wird, ggf. nach Abdestillieren flüchtiger Bestandteile (Wasser, Edukt der Formel (2)), das Halogenphenyl-oxethylsulfoxid oder -sulfon durch Filtration gewonnen, oder mit einem geeigneten Extraktionsmittel, beispielsweise Methylisobutylketon, aus dem Produktgemisch extrahiert und nach Abdampfen desselben in Form einer Schmelze isoliert.

Bei Verwendung destillativ vom Edukt der Formel (2) befreiter Verbindungen der Formel (1) mit n = 0 kann das nach der Oxidation entstandene, in der Regel wasserlösliche, Zielprodukt in seiner heißen wäßrigen Lösung, ggf. nach Zusatz eines Adsorbens, wie beispielsweise einer Klärkohle oder Kieselerde, von unlöslichen Verunreinigungen und gelösten adsorptiv entfernbaren Nebenprodukten geklärt und nach Abkühlen des Filtrats extraktiv oder durch Filtration isoliert werden.

Von besonderem Vorteil, und daher bevorzugt, ist die Oxidation von destillativ gereinigten Halogenphenyl-oxethylsulfiden der Formel (1) mit n = 0 mit Wasserstoffoperoxid, vorzugsweise in Gegenwart katalytischer Mengen Wolfram(VI)- verbindungen, weil dann nach beendeter Reaktion die Zielprodukte direkt durch Filtration oder Phasentrennung in reiner Form isoliert werden können.

Besonders bevorzugt ist demnach eine Verfahrensweise, bei der das Umsetzungsgemisch der Halogenaustausch-Stufe destillativ in wiedereinsetzbares Edukt der Formel (2) einerseits und reines Halogenphenyl-oxyethylsulfid andererseits getrennt und letzteres dann in Substanz mit der erforderlichen Menge Wasserstoffperoxid in Gegenwart katalytischer Mengen Wolfram (VI)-verbindungen bei pH 4—6 zu den Verbindungen der Formel (1) mit n = 1 oder 2 oxidiert wird. Das neue erfindungsgemäße Verfahren vermeidet die eingangs geschilderten Nachteile bekannter Synthese-Alternativen, führt überraschenderweise in guten Ausbeuten zu reinen Verbindungen der Formel (1) (mit n = 1 oder 2) und stellt somit einen erheblichen technischen Fortschritt dar.

Die Verbindungen der Formel (1) mit n = 2 stellen, wie eingangs schon erwähnt, wichtige Vorprodukte zur Herstellung von Reaktivfarbstoffen der Vinylsulfonreihe dar; die Verbindungen der Formel (1) mit n = 0 und n = 1 insofern ebenfalls, als sie durch die erfindungsgemäße Oxidation in die erstgenannten Verbindungen überführt werden können.

Die folgenden Beispiele sollen das Verfahren näher erläutern, ohne es darauf zu beschränken.

## Beispiel 1

Zu 297 Teilen 60 gewichts %iger Kalilauge läßt man unter Inertgasüberlagerung 234 Teile Mercaptoethanol so zulaufen, daß eine Innentemperatur von 35°C nicht überschritten wird. Man gibt 1000 Teile o-Dichlorbenzol und 300 Teile Polyglykol 1000 dimethylether zu, setzt den Ansatzkolben unter 350 Torr Vakuum und heizt auf eine Innentemperatur von 115—120°C. Dabei beginnt ein Gemisch aus Wasser und o-Dichlorbenzol abzudestillieren. Das als Oberphase in einem aufgesetzten Wasserabscheider sich sammelnde Wasser wird abgenommen, die o-Dichlorbenzol-Unterphase läuft in den Ansatz zurück. Man rührt insgesamt 15 Std. bei 115—120°C, wobei sich ca. 190 Teile Wasser abtrennen. Anschließend wird das nicht umgesetzte o-Dichlorbenzol (706 Teile) mit Wasserdampf abgeblasen. Es kann ohne weitere Reinigung beim nächsten Ansatz wiederverwendet werden.

Aus dem resultierenden Zweiphasensystem wird die organische Unterphase abgetrennt, mit 500 Teilen Wasser von Raumtemperatur versetzt und mit Essigsäure ein pH-Wert von 7,0 eingestellt. Es wird 30 Minuten verrührt, dann die organische Phase erneut abgetrennt und im Vakuum fraktioniert. Bei 1 Torr und 120—123°C Kopftemperatur werden 358,0 Teile Destillat erhalten, das nach Gaschromatogramm (gegen authentischen inneren Standard) 95%ig an o-Chlorphenyl-oxethylsulfid ist, so daß 340,0 Teile der Verbindung der Formel

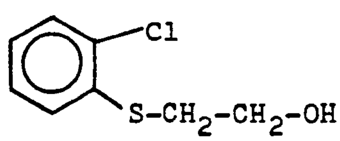

erhalten werden, was einer Ausbeute von 90,2% der Theorie, bezogen auf umgesetztes o-Dichlorbenzol, entspricht.

Ersetzt man den Polyglykol 1000 -dimethylether durch die gleiche Menge Polyglykol 1000 und arbeitet im übrigen in der angegebenen Weise, so erhält man das o-Chlorphenyl-oxethylsulfid in gleicher Qualität, aber mit ca. 7% geringerer Ausbeute.

## Beispiel 2

Zu einer Lösung von 281 Teilen Mercaptoethanol in 200 Teilen Wasser werden bei einer Innentemperatur von maximal 30°C unter Rühren portionsweise 236 Teile Ätzkali-Schuppen (88—90%ig) gegeben. Dann wird bis zur vollständigen Lösung nachgerührt. Man sezt 600 Teile p-Dichlorbenzol und 250

Teile Polyglycol 400 zu und heizt nach Evakuieren auf 350 Torr auf eine Innentemperatur von 120—125°C. Dabei beginnt ein Gemisch aus Wasser und p-Dichlorbenzol abzudestillieren, das in einem auf 55—60°C temperierten Wasserabscheider aufgefangen wird. Die wäßrige Oberphase wird abgenommen, die p-Dichlorbenzol-Unterphase läuft in den Ansatz zurück. Man rührt insgesamt 12 Stunden bei 120—125°C, wobei sich ca. 280 Teile Wasser abtrennen lassen.

Man steigert nun das Vakuum auf 4—5 Torr und destilliert bei einer Sumpftemperatur von maximal 110°C nicht umgesetztes p-Dichlorbenzol ab. Man erhält 200 Teile p-Dichlorbenzol-Destillat, das beim nächsten Ansatz wieder eingesetzt werden kann. Der verbleibende Sumpf wird über eine kurze Kolonne destilliert, wobei bei 1 Torr und 132—134°C Kopftemperatur 462,7 Teile Destillat erhalten werden, das laut gaschromatografischer Analyse (gemessen gegen authentischen inneren Standard) 94,8%ig an p-Chlorphenyloxethylsulfid ist, so daß 438,6 Teile der Verbindung der Formel

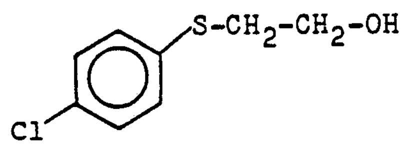

erhalten werden, was einer Ausbeute von 85,5% der Theorie, bezogen auf umgesetztes p-Dichlorbenzol, entspricht.

Ersetzt man die Ätzkali-Schuppen durch aliquote Mengen an Ätznatron-Schuppen und arbeitet im übrigen in der angegebenen Weise, so wird bei ähnlicher Reinheit eine um ca. 9% niedrigere Ausbeute erhalten.

Beispiel 3

Eine Mischung aus 300 Teilen m-Dichlorbenzol und 137,5 Teilen Mercaptoethanol wird bei 20—25°C unter Rühren und Stickstoff-Überlagerung innerhalb 30 Minuten tropfenweise mit 212 Teilen 50%iger Kalilauge versetzt. Man setzt die Apparatur unter ein Vakuum von 260—280 Torr und heizt auf 110—120°C. Innerhalb 4 Stunden lassen sich mittels aufgesetztem Wasserabscheider 145 Teile Wasser abtrennen. Das mit überdestillierende m-Dichlorbenzol läuft kontinuierlich in den Ansat zurück.

Man setzt nun 50 Teile Polyglykol 600-diethylether zu und rührt weitere 10 Stunden bei 120—125°C nach. Bei einem Vakuum von 2—5 Torr wird nun das nicht umgesetzte m-Dichlorbenzol abdestilliert (Sumpftemperatur maximal 110°C). Das Destillat (105 Teile m-Dichlorbenzol) kann ohne Vorbehandlung im nächsten Ansatz wieder eingesetzt werden.

Der verbleibende Sumpf wird über eine kurze kolonne fraktioniert. Die bei 1—2 Torr und einer Kopftemperatur von 131—134°C übergehende Fraktion (200,7 Teile) besteht zu 95,6% aus m-Chlorphenyl-oxethylsulfid (Gaschromatogramm mit authentischem innerem Standard). Demnach werden 191,9 Teile Verbindung der Formel

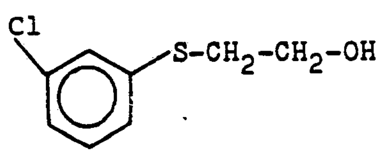

erhalten, was einer Ausbeute von 76,7% der Theorie, bezogen auf umgesetztes m-Dichlorbenzol, entspricht.

Beispiele 4—12

Arbeitet man nach dem in Beispiel 3 beschriebenen Verfahren, ersetzt aber das m-Dichlorbenzol durch aliquote Teile der in der nachstehenden Tabelle 1 aufgeführten Ausgangsprodukte, die 50%ige Kalilauge durch entsprechende Mengen der aus der Tabelle 1 ersichtlichen Alkalimetallverbindungen und den Polyglykol 600-diethylether ggf. durch den dort gewichtsmäßig angegebenen Katalysator, und arbeitet im übrigen in der angegebenen Weise, so erhält man die in der Tabelle 1 mit ihren Siedepunkten, Reingehalten und Ausbeuten aufgeführten Verbindungen der Formel (1) mit n = 0.

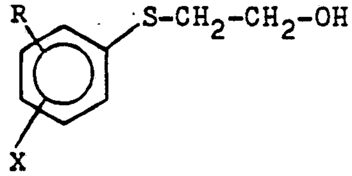

TABELLE 1

| Beispiel | Edukt | Alkali-verbindungen | Katalysator | | Produkt | | | | Ausbeute |
|---|---|---|---|---|---|---|---|---|---|
| | | | Art | Teile | R | X | Kp(°C)/Torr | RG (GC) | |
| 4 | o-Dichlorbenzol | $K_2CO_3$ | 18-Krone-6 | 9 | 2-Cl | H | 123/1 | 98,8% | 65,0% |
| 5 | o-Dichlorbenzol | Ätzkali | PEG 2000 | 100 | 2-Cl | H | 123/1 | 93,9% | 87,4% |
| 6 | o-Dichlorbenzol | LiOH | 15-Krone-5 | 6 | 4-Cl | H | 134/1 | 98,2% | 71,4% |
| 7 | p-Dichlorbenzol | Ätznatron | PEG 400-DME | 300 | 4-Cl | H | 134/1 | 91,9% | 57,3% |
| 8 | p-Dichlorbenzol | Ätzkali | PEG 1000-DME | 100 | 4-Cl | H | 134/1 | 96,2% | 89,4% |
| 9 | p-Dibrombenzol | Ätzkali | PEG 1000-DME | 70 | 4-Br | H | 141/1 | 97,9% | 91,2% |
| 10 | 3,4-Dichlortoluol | Ätzkali | PEG 1000-DME | 100 | 2-Cl | 4/5-$CH_3$ (Gemisch) | 142/1 | 91,9% | 58,9% |

PEG: Polyethylenglykol DME: -dimethylether DBE: -dibutylether (n) GC: Gaschromatogramm

Beispiel 13

Eine Emulsion aus 99 Teilen o-Chlorphenyl-oxethylsulfid (RG 95,0%, hergestellt nach Beispiel 1) und 400 Teilen Wasser wird unter Rühren mit Essigsäure auf pH 3 eingestellt und auf 50°C erwärmt. Man gibt nun in 15 Minuten 49 Teile 35%iges Wasserstoffperoxid zu, heizt auf 90°C und rührt 5—6 Stunden bei dieser Temperatur nach. Dann setzt man 5 Teile Aktivkohle zu, klärt nach 5 Minuten über ein beheiztes Filter und kühlt das Filtrat unter Rühren auf 10°C ab. Die dabei ausgeschiedenen farblosen Kristsalle werden abgesaugt, mit Kaltwasser gewaschen und im Vakuum bei 60°C getrocknet.

Man erhält 96,3 Teile o-Chlorphenyl-oxethylsulfoxid der Formel

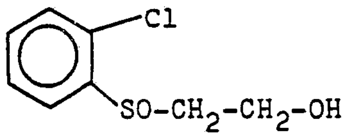

von Schmelzpunkt 111—112°C und einem Reingehalt (HPLC = high performance liquid chromatography) von 90,1%.

Die Ausbeute beträgt somit 93,6% der Theorie, bezogen auf eingesetztes o-Chlorphenyl-oxyethylsulfid.

Beispiel 14

Man wiederholt Beispiel 13, setzt aber statt 49 Teilen Wasserstoffperoxid 110 Teile Wasserstoffperoxid und 2,3 Teile Natriumwolframat zu. Das nach beendeter Reaktion kristallin isolierte Produkt wird im Vakuum bei 40°C getrocknet. Man erhält 108,1 Teile o-Chlorphenyl-oxethylsulfon der Formel

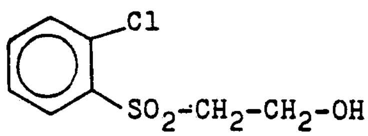

vom Schmelzpunkt 65—66°C und einem Reingehalt (HPLC) von 99,5%. Die Ausbeute errechnet sich daraus zu 97,8% der Theorie, bezogen auf eingesetztes o-Chlorphenyl-oxethylsulfid.

Beispiele 15—18

Analog Beispiel 14 lassen sich die in der nachstehenden Tabelle 2 aufgeführten Halogenphenyl-oxethylsulfone der Formel (1) mit n = 2

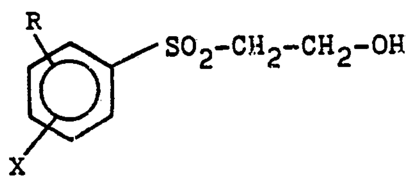

(A)

aus den entsprechenden Halogenphenyl-oxethylsulfiden der Formel (1) mit n = 0 (vgl. Tabelle 1) herstellen. Die erreichbaren Ausbeuten, sowie die Schmelzpunkte und Reingehalte (HPLC) sind aus Tabelle 2 ersichtlich.

Tabelle 2

| Beispiel | R | X | Schmelzpunkt | RG(HPLC) | Ausbeute |
|---|---|---|---|---|---|
| 15 | 3-Cl | H | 40-42° C | 98,9 % | 97,5 % |
| 16 | 4-Cl | H | 52-54° C | 99,3 % | 98,4 % |
| 17 | 4-Br | H | 50-52° C | 98,5 % | 98,0 % |

Beispiel 19

Zu einer gerührten Emulsion aus 99,4 Teilen p-Chlorphenyloxethylsulfid (RG 94,8%, hergestellt nach Beispiel 2) und 250 Teilen Wasser läßt man innerhalb 60 Minuten bei Raumtemperatur 375 Teile einer 11%igen Natriumhypochlorit-Lösung tropfen. Man rührt 2 Stunden bei Raumtemperatur nach, heizt dann zum Sieden, setzt 5 Teile aktivkohle zu und klärt nach 5 Minuten über ein beheiztes Filter. Die beim Kühlen des Filtrats auf 10°C abgeschiedenen farblosen Kristalle werden abfiltriert, mit Wasser salzfrei gewaschen und im Vakuum bei 40°C getrocknet.

Man erhält 98,2 Teile p-Chlorphenyl-oxethylsulfoxid der Formel

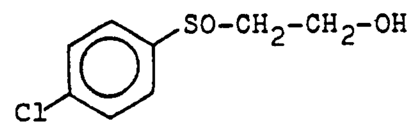

vom Schmelzpunkt 90—92°C und einem Reingehalt (HPLC) von 99,4%, was einer Ausbeute von 95,7% der Theorie, bezogen auf eingesetztes p-Chlorphenyl-oxethylsulfid, entspricht.

Beispiel 20

Man verfährt wie in Beispiel 19 beschrieben, leitet aber an Stelle der Zugabe von 375 Teilen 11%iger Natriumhypochlorit-Lösung innerhalb 2 Stunden 80 Teile Chlorgas, verdünnt mit der doppelten Menge Stickstoff, in die Emulsion ein, wobei kein Chlor aus dem Reaktionsgefäß entweichen soll.

Vor dem Hochheizen des Ansatzes zum Sieden werden zusätzlich 250 Teile Wasser zugesetzt. Die Aufarbeitung erfolgt, wie in Beispiel 19 angegeben. Man erhält 106,9 Teile p-Chlorphenyl-oxethylsulfon der Formel

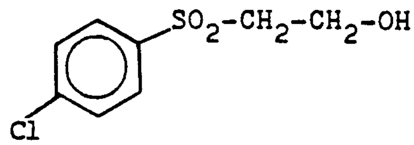

von Schmelzpunkt 53—54°C und einem Reingehalt (HPLC) von 99,4%, was einer Ausbeute von 96,6% der Theorie, bezogen auf eingesetztes p-Chlorphenyl-oxethylsulfid, entspricht.

Beispiel 21

Das gemäß Beispiel 1 nach dem Abblasen des o-Dichlorbenzols vorliegende Zweiphasen-Gemisch wird durch Zugabe von ca. 50 Teilen Eisessig auf pH 5,5 eingestellt und mit 7 Teilen Natriumwolframat versetzt. Man heizt auf 55—60°C, tropft in 70 minuten 428 Teile 35%iges Wasserstoffperoxid zu, wobei die Innentemperatur bis 95-C ansteigen darf. Man rührt 2 Stunden bei 95°C nach, kühlt auf Raumtemperatur und trennt die untere organische Schicht ab. Man erhält 350 Teile einer Produktschmelze, die nach HPLC-Analyse (gemessen gegen authentischen inneren Standard) 78,9% o-Chlorphenyl-oxethylsulfon der Formel

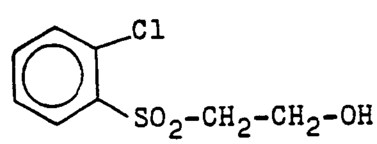

enthält, was einer Ausbeute von 276,1 Teilen, entsprechend 90,8% der Theorie, bezogen auf umgesetztes o-Dichlorbenzol, entspricht.

Das Produkt kann in der vorliegenden Form direkt zu Folgereaktionen eingesetzt werden. Beispielsweise liefert es bei der Nitrierung gemäß Beispiel 5 der DE—PS 859 462 sauberes 2-Chlor-5-nitrophenyl-oxethylsulfon von Schmelzpunkt 166—168°C und einem Reingehalt (HPLC) von 98,8% in 95%iger Ausbeute.

Beispiel 22

Das gemäß Beispiel 2 nach beendeter Reaktion vorliegende, noch nicht vom p-Dichlorbenzol befreite Umsezungsgemisch wird mit 600 Teilen Wasser vermischt. Man setzt 8 Teile Wolframtrioxid zu und stellt mit ca. 90 Teilen Eisessig einen pH-Wert von 5,5 ein.

Nun wird auf 50—55°C geheizt und innerhalb 90 Minuten durch Zulauf von 870 Teilen 35%igem Wasserstoffperoxid oxidiert, wobei die Innentemperatur auf 95°C ansteigt. Man rührt 2 Stunden bei 95°C nach, bläst das enthaltene p-Dichlorbenzol mit Wasserdampf aus (es werden 202 Teile zum Wiedereinsatz

beim nächsten Ansatz zurückgewonnen) und extrahiert die Verbleibende wäßrige Phase erschöpfend mit Methylisobutylketon (MIBK). Der Extrakt wird im Vakuum von flüchtigen Anteilenbefreit (erhalten werden 98% des eingesetzten MIBK, die im Folgeansatz erneut eingesetzt werden können). Es verbleiben 645,5 Teile einer Produktschmelze, die nach HPLC (gemessen gegen authentischen inneren Standard) 82,5% p-Chlorphenyl-oxethylsulfon der Formel

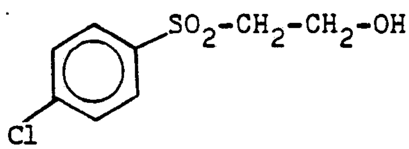

enthält, was einer Ausbeute von 532,5 Teilen, entsprechend 89,2% der Theorie, bezogen auf umgesetztes p-Dichlorbenzol, entspricht.

Die Schmelze weist einen Erstarrungspunkt von 24—26°C auf und ist in dieser Form zur Durchführung von Folgereaktionen genügend rein. So liefert sie bei Nitrierung gemäß Beispiel 5 der DE—PS 859 462 in 94,3%iger Ausbeute 4-Chlor-3-nitrophenyl-oxethylsulfon von Schmelzpunkt 95—96°C und einem Reingehalt (HPLC) von 98,9%.

**Patentansprüche**

1. Verfahren zur Herstellung von Verbindungen der allgemeinen Formel

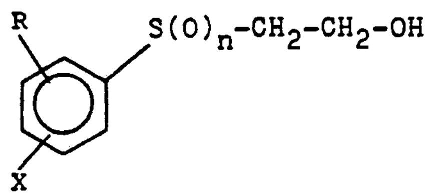

(1)

in welcher R ein Chlor- oder Bromatom, X ein Wasserstoff-, atom oder eine Alkyl-$C_1$—$C_6$-gruppe und n die Zahl 0, 1 oder 2 bedeuten, dadurch gekennzeichnet, daß man Halogenbenzole der allgemeinen Formel (2)

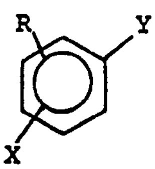

(2)

in welcher R und X die vorstehend genannten Bedeutungen haben und Y ein Chlor- oder Bromatom darstellt, mit Mercaptoethanol in Gegenwart eines Alkalimetalloxids, -hydroxids oder -carbonats und in Gegenwart von Polyglykolen, Polyglykolethern oder macrocyclischen Polyethern bei Temperaturen von 80 bis 140°C umsetzt zu den Halogenphenyl-oxyethylsulfiden der allgemeinen Formel (3)

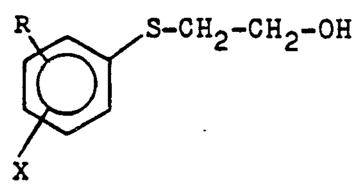

(3)

in welcher R und X die vorstehend genannten Bedeutungen haben, und diese direkt in der angefallenen Reaktionsmischung oder nach Abtrennung der flüchtigen und festen Bestandteile, vorzugsweise in Gegenwart von Wasser, bei einem pH-Wert von 1 bis 7 bei Temperaturen von 20 bis 120°C mit 1 Mol Oxidationsmittel (pro Mol Halogenphenyl-oxethylsulfid) umsetzt zu den Verbindungen der Formel (1) mit n = 1, oder mit mindestens 2 Mol Oxidationsmittel (pro Mol Halogenphenyl-oxethylsulfid) umsetzt zu den Verbindungen der Formel (1) mit n = 2.

2. Verfahren nach Anspruch 1, dadurch gekennzeichnet, daß man die Umsetzung der Verbindungen der Formel (2) mit Mercaptoethanol in Gegenwart von offenkettigen linearen Polymeren des Ethylenoxids oder deren Alkylethern der Summenformel

$$R_1—(OCH_2—CH_2)_n—OR_1$$

in welcher $R_1$ ein Wasserstoffatom oder eine Alkyl $C_1—C_4$-gruppe, und n die Zahl 3—500 bedeutet, vornimmt.

3. Verfahren nach Anspruch 1, dadurch gekennzeichnet, daß man die Umsetzung der Verbindungen der Formel (2) mit Mercaptoethanol in Gegenwart von cyclischen Polymeren des Ethylenoxids der Summenformel

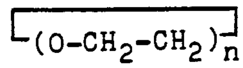

in welcher n eine Zahl von 4 bis 8 bedeutet, vornimmt.

4. Verfahren nach Anspruch 1, dadurch gekennzeichnet, daß man die Oxidation der Verbindungen der Formel (3) mit Chlor, Brom, Alkalimetallhypochlorit oder -hypobromit oder Wasserstoffperoxid vornimmt.

5. Verfahren nach Anspruch 1, dadurch gekennzeichnet, daß man die Oxidation der Verbindungen der Formel (3) mit Wasserstoffperoxid in Gegenwart von Wolframtrioxid oder Alkalimetall-Wolframat als Katalysatoren vornimmt.

**Revendications**

1. Procédé pour préparer des composés répondant à la formule générale:

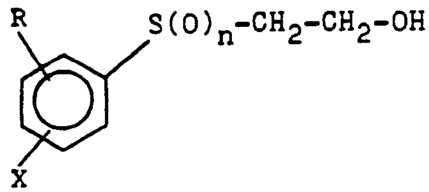

(1)

dans laquelle R représente un atome de chlore ou de brome, X un atome d'hydrogène ou un radical alkyle contenant de 1 à 6 atomes de carbone et n un nombre égal a 0, à 1 ou à 2, procédé caractérisé en ce qu'on fait réagir, à des températures de 80 à 140°C, des halogénobenzènes répondant à la formule générale 2:

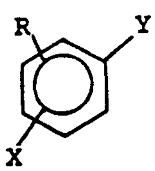

(2)

dans laquelle R et X ont les significations qui leur ont été données ci-dessus et Y représente un atome de chlore ou de brome, avec le mercapto-éthanol, en présence d'un oxyde, d'un hydroxyde ou d'un carbonate de métal alcalin et en présence de polyglycols, d'éthers de polyglycols ou de polyéthers macrocycliques, de manière à obtenir les (hydroxyéthylthio)halogénobenzènes répondant à la formule générale 3:

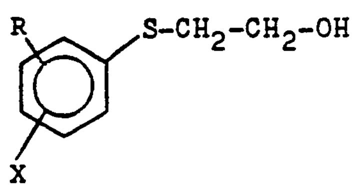

(3)

dans laquelle R et X ont les significations qui leur ont été données ci-dessus, et on fait réagir ceux-ci, soit directement dans le mélange réactionnel obtenu, soit après en avoir éliminé les constituants volatils et solides, de préférence en présence d'eau, à un pH de 1 à 7 et à des températures de 20 à 120°C, avec 1 mol d'un agent d'oxydation [par mole de l'(hydroxyéthylthio)-halogénobenzène] si l'on veut obtenir les composés de formule 1 pour lesquels n est égal à 1, ou avec au moins 2 mol d'un agent d'oxydation [par mole de l'(hydroxyéthylthio)-halogénobenzène] si l'on veut obtenir les composés de formule 1 pour lesquels n est égal à 2.

2. Procédé selon la revendication 1 caractérisé en ce que la réaction des composés de formule 2 avec le mercapto-éthanol est effectuée en présence de polymères linéaires à chaîne ouverte de l'oxyde d'éthylène ou d'éthers alkyliques de ceux-ci qui répondent à la formule brute:

$$R_1—(OCH_2—CH_2)_n—OR_1$$

dans laquelle $R_1$ représente un atome d'hydrogène ou un radical alkyle en $C_1—C_5$ et n désigne un nombre de 3 à 500.

3. Procédé selon la revendication 1 caractérisé en ce que la réaction des composés de formule 2 avec le mercapto-éthanol est effectuée en présence de polymères cycliques de l'oxyde d'éthylène répondant à la formule brute:

$$\left[ (O-CH_2-CH_2)_n \right]$$

dans laquelle n désigne un nombre de 4 à 8.

4. Procédé selon la revendication 1 caractérisé en ce que l'oxydation des composés de formule 3 est effectuée au moyen du chlore, du brome, d'un hypochlorite ou d'un hypobromite de métal alcalin, ou du peroxyde d'hydrogène.

5. Procédé selon la revendication 1 caractérisé en ce que l'oxydation des composés de formule 3 est effectuée au moyen du peroxyde d'hydrogène en présence de trioxyde de tungstène ou d'un tungstate de métal alcalin comme catalyseur.

**Claims**

1. A process for the preparation of compounds of the general formula

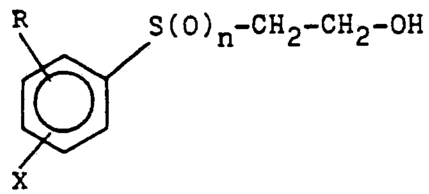

(1)

in which R denotes a chlorine or bromine atom and X denotes a hydrogen atom or a ($C_1$—$C_6$) alkyl group and n denotes the numbers 0,1 or 2, which comprises reacting halobenzenes of the general formula (2)

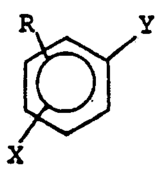

(2)

in which R and X have the meanings specified above and Y represents a chlorine or bromine atom, with mercaptoethanol in the presence of an alkali metal oxide, hydroxide or carbonate and in the presence of polyglycols, polyglycol ethers or macrocyclic polyethers at temperatures of 80—140°C to form halophenyl hydroxyethyl sulfides of the general formula (3)

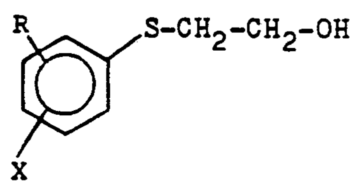

(3)

in which R and X have the meanings specified above, and reacting these directly in the reaction mixture produced or after removal of the volatile and solid components, preferably in the presence of water, at a pH of 1 to 7 at temperatures of 20—120°C with 1 mol of oxidizing agent (per mol of halophenyl hydroxyethyl sulfide) to form compounds of the formula (1) with n = 1, or reacting them with at least 2 mol of oxidizing agent (per mol of halophenyl hydroxyethyl sulfide) to form the compounds of the formula (1) with n = 2.

2. The process as claimed in claim 1, wherein the reaction of the compounds of the formula (2) with mercaptoethanol is carried out in the presence of open-chain linear polymers of ethylene oxide or alkyl ethers thereof of the total molecular formula

$$R_1—(OCH_2—CH_2)_n—OR_1$$

in which $R_1$ is a hydrogen atom or a ($C_1$—$C_4$) alkyl group and n denotes the number 3—500.

3. The process as claimed in claim 1, wherein the reaction of the compounds of the formula (2) with

mercaptoethanol is carried out in the presence of cyclic polymers of ethylene oxide of the total molecular formula

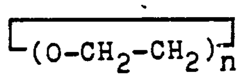

in which n denotes a number from 4 to 8.

4. The process as claimed in claim 1, wherein the oxidation of the compounds of the formula (3) is carried out with chlorine, bromine, alkali metal hypochlorite or hypobromite or hydrogen peroxide.

5. The process as claimed in claim 1, wherein the oxidation of the compounds of the formula (3) with hydrogen peroxide is carrried out in the presence of tungsten trioxide or alkali metal tungstate as catalyst.